# EUROPÄISCHE PATENTSCHRIFT

(11) **EP 2 349 354 B1**
(45) Veröffentlichungstag und Bekanntmachung des Hinweises auf die Patenterteilung: **02.09.2015**
(21) Anmeldenummer: 09783490.7
(22) Anmeldetag: 28.09.2009
(51) Int. Cl.: A61L 2/12, A61L 2/22, B29C 49/00

(54) **VERFAHREN ZUR VORBEHANDLUNG VON VORFORMLINGEN UND STRECKBLASMASCHINE ZUR VORBEHANDLUNG UND ZUM STRECKBLASEN VON VORFORMLINGEN ZU BEHÄLTERN**
METHOD FOR PRE-TREATING PREFORMS AND STRETCH BLOW MOLDING MACHINE FOR PRE-TREATING AND FOR STRETCH BLOW MOLDING OF PREFORMS INTO CONTAINERS
PROCÉDÉ DE PRÉTRAITEMENT DE PRÉ-ÉBAUCHES ET MACHINE D'ÉTIRAGE-GONFLAGE POUR LE PRÉTRAITEMENT ET L'ÉTIRAGE-GONFLAGE DE PRÉ-ÉBAUCHES POUR OBTENIR DES CONTENEURS

(30) Priorität: 07.11.2008 DE 102008056346
(43) Veröffentlichungstag der Anmeldung: 03.08.2011
(73) Patentinhaber: Krones AG, 93073 Neutraubling (DE)
(72) Erfinder: HUMELE, Heinz, 93073 Neutraubling (DE)
(74) Vertreter: Benninger, Johannes
(86) Internationale Anmeldenummer: PCT/EP2009/062532
(87) Internationale Veröffentlichungsnummer: WO 2010/052068

(56) Entgegenhaltungen:
- WO-A1-2007/140883
- WO-A1-2008/055685
- WO-A2-2007/131701
- DE-A1- 2 908 086
- US-B1- 6 984 360

## Beschreibung

Die vorliegende Erfindung betrifft ein Verfahren zur Vorbehandlung von Vorformlingen mit den Merkmalen des unabhängigen Anspruchs 1. Die Erfindung betrifft weiterhin eine Blasmaschine zur Vorbehandlung und zum Blasen von Vorformlingen zu Behältern.

Bei der Herstellung von Getränkebehältern mittels eines Blasverfahrens werden sog. Vorformlinge zunächst auf eine ausreichende Temperatur erwärmt und anschließend in eine Form eingespannten Zustand durch kontrolliertes Einblasen von Luft in eine gewünschte Behälterform gebracht. Erst wenn sie diese Behälterform haben, weisen sie das notwendige Volumen auf, um sie mit Flüssigkeit befüllen zu können. Vor dem Befüllen müssen die Behälter in der Regel sterilisiert werden, damit das abgefüllte Getränk möglichst lange haltbar bleibt. Zudem können bei ausreichend guter Sterilisierung die Zugaben von Konservierungsmitteln in den Getränken reduziert werden.

Aufgrund der zunehmend höheren Anforderungen an die Reinheit der Behälter und aufgrund einer wünschenswerten Minimierung der in den Getränken befindlichen Konservierungsstoffe sind Verfahren entwickelt worden, die eine Sterilisierung der Vorformlinge nach ihrer Erwärmung und vor dem Blasvorgang vorsehen. Auf diese Weise kann ggf. ein weitgehend steriler Prozess bis zur Abfüllung erreicht und aufrechterhalten werden.

Die EP 1 056 481 B1 beschreibt ein Verfahren zur Sterilisation von Hohlkörpern, bei dem auf die zu sterilisierenden Flächen ein zuvor verdampftes Sterilisationsmittel aufgebracht wird. Die Verdampfung des Sterilisationsmittels erfolgt außerhalb des Hohlkörpers in der Nähe seiner Öffnung. Durch Einbringen und Absaugung des Sterilisationsmittels wird innerhalb des Hohlkörpers ein Gasstrom erzeugt, um das Mittel auf alle Flächen zu verteilen. Der Hohlkörper kann entweder ein bereits in Form gebrachter Getränkebehälter oder ein Vorformling sein, der anschließend zu einem Getränkebehälter umgeformt wird.

Die JP 04 04 49 02 A beschreibt ein Verfahren zum Sterilisieren von Vorformlingen, bevor diese erwärmt und anschließend durch Streckblasen in die gewünschte Form eines Getränkebehälters gebracht werden.

Bisher war es üblich, die Vorformlinge mittels Heißluft und/oder durch Infrarotbestrahlung auf die für das Blasformen notwendige Temperatur zu bringen. Aus der WO 2007/131701 A2 ist eine Erwärmungsvorrichtung für Kunststoffrohlinge bekannt, die mit Mikrowellenstrahlung arbeitet.

Die WO 2008/055 685 A1 offenbart eine Vorrichtung zur Herstellung von Getränkeflaschen. Hierbei werden verschiedene Möglichkeiten aufgezeigt, wie alternative Behandlungsmethoden für verschiedene Flaschenarten angewendet werden können. Die Vorrichtung weist hierfür verschiedene Aggregate, beispielsweise Kühl- und/oder Wärme- und/oder Sterilisationsaggregate auf. Im Sterilisationsaggregat wird der Behälter mittels Sterilisationsmittel beaufschlagt.

Die WO 2007/140883 A1 offenbart ein Verfahren zum Sterilisieren von hitzeempfindlichen Flaschen, wobei das Sterilisationsmittel als Aerosol eingebracht und mittels eines Energieeintrags aktiviert wird.

Die WO 2007/131 701 A2 offenbart eine Vorrichtung zum Erhitzen von Preforms bei der Flaschenherstellung. Gemäß Offenbarung der WO-Schrift wird der Preform soweit erhitzt, dass dieser anschließend in eine gewünschte Form geblasen werden kann.

Die DE 29 08 086 A1 offenbart eine Sterilisierungsmethode von Krankenhausbetten, die in einer Desinfektionskammer mit Desinfektionsmittel bedampft werden. Über Mikrowellen wird anschließend das Desinfektionsmittel aktiviert.

Ein Ziel der vorliegenden Erfindung besteht darin, eine möglichst schnelle und einfache Temperierung der Vorformlinge sowie eine damit im Zusammenhang stehende Sterilisierung der Teile zu erreichen, um damit einen weitgehend geschlossenen Prozess für eine nachfolgende Umformung der Vorformlinge zu Behältern und deren Befüllung mit Flüssigkeit zu ermöglichen.

Dieses Ziel der Erfindung wird mit den Gegenständen der unabhängigen Ansprüche erreicht. Merkmale vorteilhafter Weiterbildungen der Erfindung ergeben sich aus den jeweiligen abhängigen Ansprüchen.

Die Erfindung umfasst ein Verfahren zur Vorbehandlung von Vorformlingen aus thermoplastischem Kunststoff, bevor diese mittels eines Blasverfahrens zu Behältern umgeformt und mit Flüssigkeit befüllt werden. Bei dem Verfahren werden die Vorformlinge für das nachfolgende Blasverfahren temperiert und sterilisiert. Die Temperierung bzw. Erwärmung der Vorformlinge erfolgt mittels einer Bestrahlung mit Mikrowellen. Die Sterilisation der Vorformlinge erfolgt während der Vorbehandlung und mittels Einbringen eines flüssigen und/oder gasförmigen Sterilisationsmediums in die Vorformlinge.

Gemäß einer bevorzugten Ausführungsform der Erfindung wird der Behälter mittels eines Streckblasverfahrens in einer Streckblasmaschine hergestellt. Die Streckblasmaschine weist im Gegensatz zur Blasmaschine zusätzlich eine sog. Reckstange auf, die den Behälter in etwa zeitgleich zum Blasvorgang in axialer Richtung streckt. Dies hat eine biaxiale Orientierung des fertigen Behälters zur Folge, was sich insgesamt in einer höheren Stabilität der Behälter niederschlägt und für vor allem Kohlensäurehaltige Getränkebehälter von Vorteil ist.

Von besonderem Vorteil ist es, wenn die Sterilisation der Vorformlinge während der Mikrowellenbestrahlung erfolgt, da auf diese Weise ein kontinuierlicher und vollautomatischer Prozess ermöglicht wird, bei dem die Prozessschritte des Temperierens und Sterilisierens der Behälter gleichzeitig oder sich zeitlich überschneidend ablaufen können. Es war bereits bekannt, dass die Vorformlinge sich mittels einer Mikrowellenbestrahlung auf eine passende Temperatur für ein Streckblasverfahren erwärmen lassen. Im Zusammenhang mit der vorliegenden Erfindung wurde nun erkannt, dass die Beaufschlagung der Vorformlinge während der Mikrowellenbehandlung mit einem geeigneten Fluid, Gas, Flüssigkeit oder Gas-Flüssigkeitsgemisch Vorteile hinsichtlich der Wechselwirkung der Mikrowellenstrahlung mit dem thermoplastischen Kunststoff der Vorformlinge mit sich bringen kann. Dieser Effekt kann besonders vorteilhaft dazu genutzt werden, die Vorformlinge bereits während der Erwärmung zu sterilisieren, indem als Fluid ein geeignetes Sterilisationsmedium verwendet wird, das einerseits durch die Mikrowellenbestrahlung aktiviert und in seiner sterilisierenden Wirkung verstärkt werden kann. Andererseits kann das gezielt in den Vorformling eingebrachte Fluid in vorteilhafter Weise mit der Mikrowellenstrahlung in Wechselwirkung treten, so dass der Effekt der Strahlung verstärkt und die Vorformlinge schneller und/oder mit geringerer Strahlungsintensität auf die gewünschte Temperatur gebracht werden können.

Als Sterilisationsmedium kann bspw. flüssiges und/oder gasförmiges Wasserstoffperoxid (H2O2), Peressigsäure o. dgl. verwendet werden. Das Sterilisationsmedium kann in flüssiger und/oder gasförmiger Form in den Vorformling eingebracht werden. So kann als Sterilisationsmedium ein Gemisch aus Heißluft und Wasserstoffperoxid, Peressigsäure o. dgl. eingesetzt werden. Grundsätzlich können auch andere Sterilisationsmedien verwendet werden, welche eine ausreichende Wirkung aufweisen. Von Vorteil ist es, wenn das Sterilisationsmedium die gewünschte Wechselwirkung mit der Mikrowellenstrahlung zeigt, so dass sowohl die Bestrahlung effektiver wirkt und/oder die Erwärmung der Vorformlinge unterstützt wird.

Bei einer bevorzugten Variante des erfindungsgemäßen Verfahrens wird das Sterilisationsmedium mittels jeweils wenigstens einer Düse in den Vorformling eingebracht, so dass es dort schnell eingebracht und weitgehend homogen verteilt werden kann. Die Düse ist vorzugsweise im Halter bzw. im Haltedorn für den Vorformling angeordnet. Wahlweise kann eine zusätzliche Absaugeinrichtung vorgesehen sein, die nach dem Einbringen des Sterilisationsmediums und nach der Bestrahlung des Vorformlings für eine schnelle und rückstandsfreie Absaugung des Sterilisationsmediums aus den Vorformlingen sorgen kann.

Zudem wird das Sterilisationsmedium mittels der Mikrowellenbestrahlung verdampft wird und entwickelt an der Oberfläche der Vorformlinge seine sterilisierende Wirkung. Weiterhin können die Vorformlinge während des Erwärmens oder nach dem Erwärmen, nach der Sterilisationsbehandlung mittels eines geeigneten Spülmediums durchgespült und dabei von Spuren des Sterilisationsmediums befreit werden.

Die Erwärmung und Sterilisation der Vorformlinge kann insbesondere in einem kontinuierlichen Prozess, insbesondere auf einer Rundläufermaschine, erfolgen.

Die vorliegende Erfindung umfasst weiterhin eine Streckblasmaschine zur Vorbehandlung und zum Streckblasen von Vorformlingen aus thermoplastischem Kunststoff zu Behältern. Die Maschine weist Handhabungseinrichtungen zur Handhabung und kontinuierlichen Behandlung und Beförderung der Vorformlinge, eine Temperiereinrichtung zur Erwärmung der Vorformlinge sowie eine Sterilisationseinrichtung auf, wobei die Temperiereinrichtung eine Mikrowellenbestrahlungseinheit umfasst. Zudem ist die Sterilisationseinrichtung zumindest teilweise in die Handhabungseinrichtung integriert, so dass die Sterilisation der Behälter im Zusammenhang und/oder in vorteilhafter Kombination mit der Mikrowellenbestrahlung und der damit bewirkten Erwärmung der Vorformlinge erfolgen kann.

Auch weisen die Handhabungseinrichtungen jeweils Haltedorne zum Halten der Vorformlinge an deren Halsbereich auf, die mit Düsen zum Einbringen eines Sterilisationsmediums versehen sind. Zudem können die Düsen der Haltedorne jeweils über Zu- und/oder Ableitungen mit Sterilisationsmedium beaufschlagbar sein, das nach der Behandlung wieder absaugbar ist, ggf. ebenfalls mittels der Düsen oder durch andere geeignete Absaugeinrichtungen.

Die erfindungsgemäße Maschine kann insbesondere als Rundläufermaschine ausgebildet sein, in der die Vorformlinge kontinuierlich zu sterilisierten Behältern umformbar sind.

Wenn im vorliegenden Zusammenhang von Vorformlingen die Rede ist, so ist damit generell das Vorprodukt eines Behälters gemeint, der insbesondere zur Aufnahme von Getränken dienen kann. Die Vorformlinge werden teilweise auch als Rohlinge bezeichnet. Es handelt sich dabei um einseitig geschlossene Röhrchen, die an ihrem offenen Ende einen Halsbereich aufweisen, mit dem sie in eine Form eingespannt werden, in der sie mittels eines Streckblasverfahrens zu Behältern umgeformt werden. Die Vorformlinge bestehen aus einem geeigneten thermoplastischen Kunststoff, bspw. aus PET (Polyethylen).

Weitere Merkmale, Ziele und Vorteile der vorliegenden Erfindung gehen aus der nun folgenden detaillierten Beschreibung einer bevorzugten Ausführungsform der Erfindung hervor, die als nicht einschränkendes Beispiel dient und auf die beigefügten Zeichnungen Bezug nimmt. Gleiche Bauteile weisen dabei grundsätzlich gleiche Bezugszeichen auf und werden teilweise nicht mehrfach erläutert.
Fig. 1 zeigt eine schematische Darstellung einer Temperier- und Sterilisationseinrichtung.
Fig. 2 zeigt einen Querschnitt einer Aufnahmeeinrichtung für einen Vorformling.
Fig. 3 zeigt einen vergrößerten Ausschnitt der Aufnahmeeinrichtung für einen Vorformling gemäß Fig. 3.
Fig. 4 zeigt eine Draufsicht auf eine Anlage zum Herstellen von Behältern.

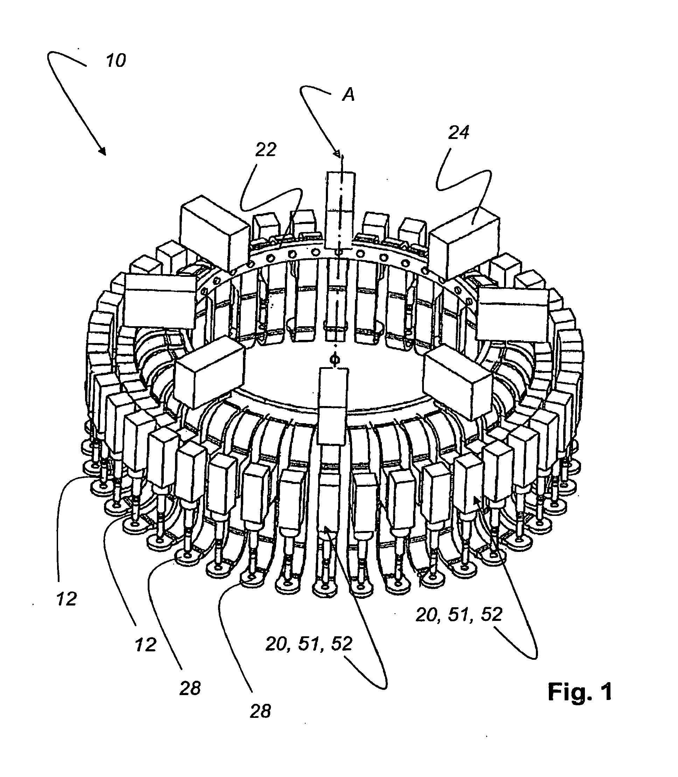

Die schematische Darstellung der Fig. 1 zeigt eine Handhabungseinrichtung 10 zum Behandeln von Vorformlingen 12. Die Handhabungseinrichtung 10 weist eine Temperiereinrichtung 51 und eine Sterilisationseinrichtung 52 auf, die in einzelnen Behandlungsstationen 20 integriert sind. Die Vorformlinge 12 werden für das Verfahren einem kontinuierlichen Prozess zugeordnet, wobei diese Vorformlinge 12 im Laufe der Erwärmung durch die Temperiereinrichtung 51 und gleichzeitiger Sterilisation durch die Sterilisationseinrichtung 52 auf einer Kreisbahn bewegt werden können. Die Behandlungsstationen 20 sind miteinander an einem Träger 22 befestigt. Zudem können an diesem Träger 22 noch weitere Bauteile angebracht werden, bspw. sind in diesem Ausführungsbeispiel acht Mikrowellenbestrahlungseinheiten 24 angeschlossen. Die mögliche Funktionsweise einer solchen Einheit kann bspw. der WO 2007/131701 A2 entnommen werden. Die Temperiereinrichtungen 51 sind mit diesen Mikrowellenbestrahlungseinheiten 24 verbunden und laufen während der Behandlung der Vorformlinge 12 gemeinsam um eine Maschinenachse A sowie die Sterilisationseinrichtung 52.

In diesem Ausführungsbeispiel wird, wie eben schon erwähnt der Vorformling 12 mittels der Temperiereinrichtung 51 und der Mikrowellenbestrahlungseinheit 24 auf eine gewünschte Temperatur gebracht. Um von einer gleichmäßigen Erwärmung des Vorformlings 12 ausgehen zu können, wird der Vorformling 12 durch einen Resonator 28, der wiederum mit der Temperiereinrichtung 51 verbunden ist, geschoben. Durch eine Sterilisationseinrichtung 52, die zumindest teilweise in die Handhabungseinrichtung 10 integriert ist, wird die Sterilisation der Vorformlinge 12 im Zusammenhang und/oder in vorteilhafter Kombination mit der Mikrowellenbestrahlungseinheit 24 und der damit bewirkten Erwärmung der Vorformlinge 12 durchgeführt.

Die Übergabe der Vorformlinge 12 an die Handhabungseinrichtung 10 kann mittels einer vorgeschalteten Einheit erfolgen, die bspw. als ein Stern, wie z.B. einem Sägezahnstern oder einem Klammerstern (nicht dargestellt) ausgebildet ist.

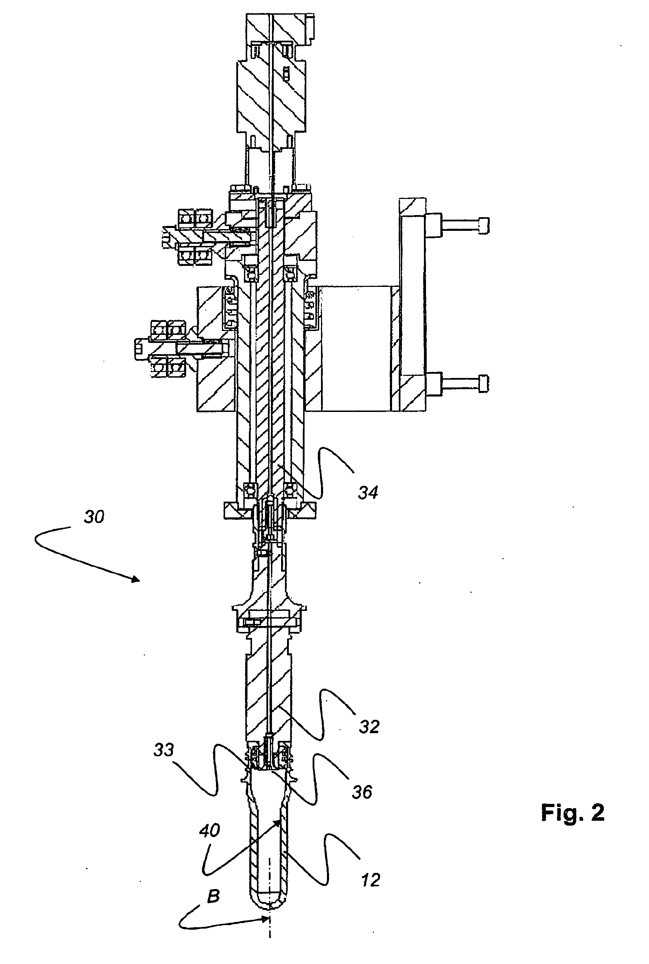

Die Figur 2 zeigt einen Querschnitt einer Aufnahmeeinrichtung 30 für einen Vorformling 12. Die Aufnahmeeinrichtung 30 umfasst eine Vorformlingshalteeinheit 32 und eine Bewegungseinheit 34. Im gezeigten Ausführungsbeispiel ist die Vorformlingshalteeinheit 32 als Haltedom ausgebildet, der in die Mündung des Halsbereiches 33 des Vorformlings 12 eindringt und diesen somit hält. Die Bewegungseinheit 34 wird vorzugsweise von einem Antrieb gebildet, der alle Bewegungserfordernisse erfüllt. Zum einen ist eine Senkbewegung erforderlich, die den Vorformling 12 entlang seiner Längsachse B von oben in den Resonator 28 (in Fig. 1 dargestellt) einführt. Zum anderen ist auch eine Hubbwegung erforderlich, die den Vorformling 12 entlang seiner Längsachse B wieder aus dem Resonator 28 (in Fig. 1 dargestellt) herausführt. Die Vorformlingshalteeinheit 32 umfasst weiterhin eine Düse 36, die zum Einbringen eines flüssigen und/oder gasförmigen Sterilisationsmediums in den Vorformling 12 dient. Das Sterilisationsmedium wird während der Mikrowellenbestrahlung in den Vorformling 12 eingebracht und verdampft durch die Erwärmung des Vorformlings 12. Dieses Sterilisationsmedium entwickelt somit an der Oberfläche 40 der Vorformlinge 12 seine sterilisierende Wirkung. In diesem Ausführungsbeispiel ist die Sterilisationseinrichtung 52 mit der Aufnahmeeinrichtung 30 und zugleich mit der Temperiereinrichtung 51 verbunden. Wahlweise kann die Sterilisation auch nach dem Erwärmen des Vorformlings 12 durchgeführt werden.

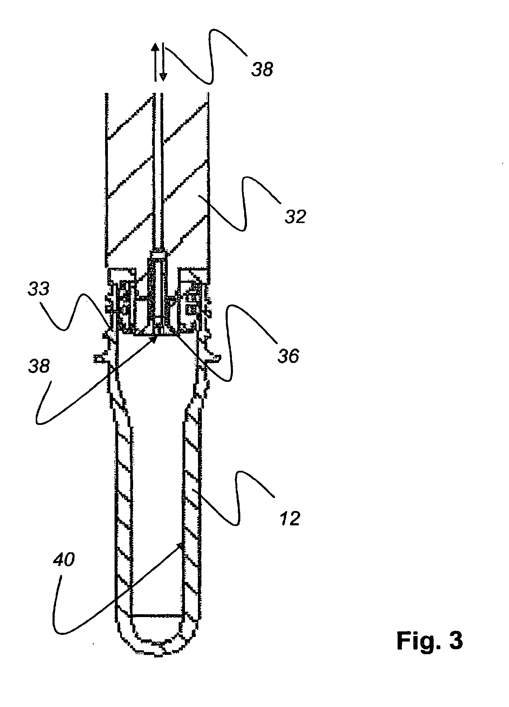

Die Figur 3 zeigt einen vergrößerten Ausschnitt einer möglichen Sterilisationseinrichtung 52 für einen Vorformling 12 gemäß Figur 2. Aus dieser Figur 3 ist die Aufnahmeeinrichtung 30 des Vorformlings 12 nochmals deutlich dargestellt. Mit der Vorformlingshalteeinheit 32, die in die Mündung des Halsbereichs 33 des Vorformlings 12 eindringt, wird der Vorformling 12 während seiner Vorbehandlung gehalten. Die Düse 36 kann jeweils über Zu- und/oder Ableitungen 38 mit dem Sterilisationsmedium beaufschlagt und/oder dieses aus dem Vorformling 12 abgesaugt werden, um an der Oberfläche 40 des Vorformlings 12 durch zusätzliche Erwärmung seine sterilisierende Wirkung zu entwickeln.

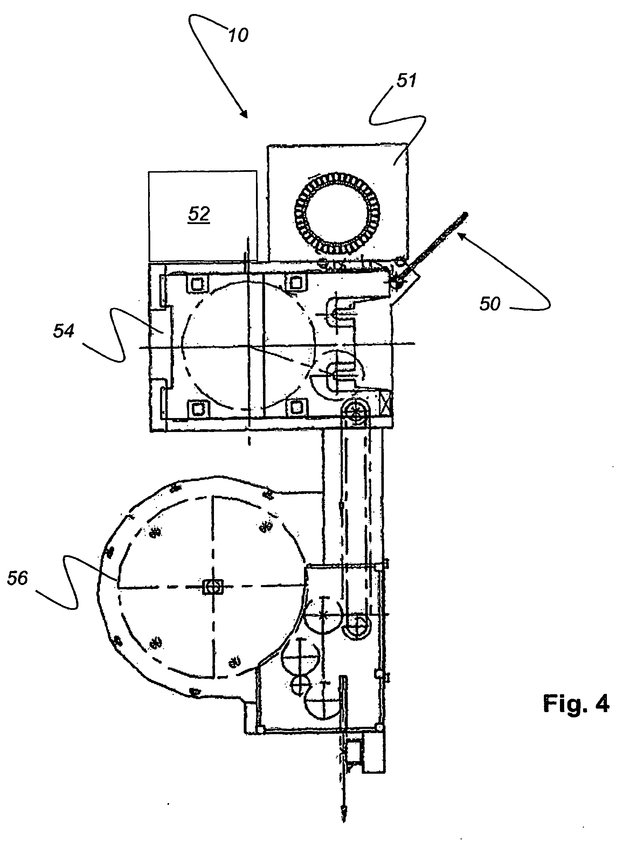

Die Figur 4 zeigt eine weitere Ausführungsform der Anordnung der Behandlungsstationen und der Vorrichtung zum Herstellen von Behältern. Über eine Zuführung 50 werden die Vorformlinge 12 an Handhabungseinrichtungen 10 übergeben. Die Vorformlinge 12 werden an einzelne Temperiereinrichtungen 51, die mit einer Mikrowellenbestrahlungseinheit 24 verbunden sind, übergeben. Hier werden die Vorformlinge 12 mittels Mikrowellenstrahlen erwärmt und unmittelbar nach ihrer Erwärmung einer nachgeordneten Sterilisationseinrichtung 52 übergeben. In dieser Sterilisationseinrichtung 52 werden die Vorformlinge 12 sterilisiert und ggf. nach der Sterilisierung gespült. Anschließend erfolgt eine Übergabe der vorbehandelten Vorformlinge 12 mittels einer Sternanordnung an eine Streckblasmaschine 54. Die Streckblasmaschine 54 stellt aus den Vorformlingen 12 fertige Behälter her. Nach der Behälterherstellung kann eine Übergabe an eine Füllmaschine 56 und anschließend an einen Verschließer (nicht dargestellt) und/oder einer Etikettiermaschine (nicht dargestellt) erfolgen.

## Patentansprüche

1. Verfahren zur Vorbehandlung von Vorformlingen (12) aus thermoplastischem Kunststoff, bevor diese mittels eines Blasverfahrens zu Behältern umgeformt werden, bei welchem Verfahren die Vorformlinge (12) für das nachfolgende Blasverfahren temperiert und sterilisiert werden, wobei eine Temperierung der Vorformlinge (12) mittels einer Behandlung mit Mikrowellen erfolgt, **gekennzeichnet durch** eine Sterilisation der Vorformlinge mittels Einbringen eines flüssigen und/oder gasförmigen Sterilisationsmediums, wobei die Sterilisation der Vorformlinge (12) vor und/oder während der Mikrowellenbestrahlung erfolgt und das Sterilisationsmedium mittels der Mikrowellenbestrahlung verdampft wird.

2. Verfahren nach Anspruch 1, **dadurch gekennzeichnet, dass** das Sterilisationsmedium flüssiges und/oder gasförmiges Wasserstoffperoxid (H2O2) und/oder Peressigsäure umfasst.

3. Verfahren nach einem der Ansprüche 1 oder 2, **dadurch gekennzeichnet, dass** als Sterilisationsmedium ein Gemisch aus Heißluft und Wasserstoffperoxid und/oder Peressigsäure eingesetzt wird.

4. Verfahren nach einem der Ansprüche 1 bis 3, **dadurch gekennzeichnet, dass** das Sterilisationsmedium in den Vorformling (12) mittels jeweils wenigstens einer Düse (36) eingebracht wird.

5. Verfahren nach einem der Ansprüche 1 bis 4, **dadurch gekennzeichnet, dass** die Vorformlinge (12) mittels eines geeigneten Spülmediums durchgespült und dabei von Spuren des Sterilisationsmediums befreit werden.

6. Verfahren nach einem der Ansprüche 1 bis 5, **dadurch gekennzeichnet, dass** die Erwärmung und Sterilisation der Vorformlinge (12) in einem kontinuierlichen Prozess, insbesondere auf einer Rundläufermaschine, erfolgt.

7. Verfahren nach einem oder mehreren der Ansprüche 1 bis 6, bei welchem der Vorformling (12) während seiner Vorbehandlung mit einer Vorformlingshalteeinheit (32), die in eine Mündung eines Halsbereichs (33) des Vorformlings (12) eindringt, gehalten wird.

8. Blasmaschine (54) zur Vorbehandlung und zum Blasen von Vorformlingen (12) aus thermoplastischem Kunststoff zu Behältern nach einem Verfahren gemäß der Ansprüche 1 bis 7, mit Handhabungseinrichtungen (10) zur Handhabung und kontinuierlichen Behandlung und Beförderung der Vorformlinge (12), mit einer Temperiereinrichtung (51) zur Erwärmung der Vorformlinge (12) und mit einer Sterilisationseinrichtung (52), wobei die Temperiereinrichtung (51) eine Mikrowellenbestrahlungseinheit (24) umfasst und die Sterilisationseinrichtung (52) zumindest teilweise in die Handhabungseinrichtung (10) integriert ist, **dadurch gekennzeichnet, dass** die Handhabungseinrichtungen (10) jeweils Haltedorne (32) zum Halten der Vorformlinge (12) an deren Halsbereich (33) aufweisen, die mit Düsen (36) zum Einbringen eines Sterilisationsmediums versehen sind.

9. Blasmaschine (54) nach Anspruch 8, **dadurch gekennzeichnet, dass** die Düsen (36) der Haltedorne (32) jeweils über Zu- und/oder Ableitungen (38) mit Sterilisationsmedium beaufschlagbar sind, und dass dieses absaugbar ist.

10. Blasmaschine (54) nach einem der Ansprüche 8 bis 9, **dadurch gekennzeichnet, dass** die Blasmaschine (54) als Rundläufermaschine und/oder als Streckblasmaschine ausgebildet ist, in der die Vorformlinge (12) kontinuierlich zu sterilisierten Behältern umformbar sind.

## Claims

1. A method for pretreating preforms (12), which are made of thermoplastic material, before the preforms (12) are formed into containers by means of a blow moulding process, in which method the preforms (12) are tempered and sterilised for the subsequent blow moulding process, a tempering of the preforms (12) here being carried out by means of a treatment with microwaves, **characterised by** a sterilisation of the preforms by means of introducing a liquid and/or gaseous sterilising medium, wherein the sterilisation of the preforms (12) is carried out prior to and/or during the irradiation with microwaves and the sterilising medium is evaporated by means of the irradiation with microwaves.

2. The method as recited in claim 1, **characterised in that** the sterilising medium comprises liquid and/or gaseous hydrogen peroxide (H2O2) and/or peracetic acid.

3. The method as recited in one of the claims 1 or 2, **characterised in that** a mixture of hot air and hydrogen peroxide and/or peracetic acid is used as a sterilising medium.

4. The method as recited in one of the claims 1 to 3, **characterised in that** the sterilising medium is introduced into the preform (12) by means of at least one nozzle (36), respectively.

5. The method as recited in one of the claims 1 to 4, **characterised in that** the preforms (12) are rinsed through by means of a suitable rinsing medium and thus freed from traces of the sterilising medium.

6. The method as recited in one of the claims 1 to 5, **characterised in that** the heating and sterilisation of the preforms (12) is carried out in a continuous process, in particular on a rotary machine.

7. The method as recited in one or more of the claims 1 to 6 wherein the preform (12) is held by a preform holding unit (32), which enters into the mouth of a neck section (33) of the preform (12), during the pretreatment of the preform (12).

8. A blow moulding machine (54) for pretreating and for blowing preforms (12), which are made of thermoplastic material, to form containers according to a method as recited in the claims 1 to 7, said blow moulding machine (54) having handling devices (10) for handling and continuously treating and conveying the preforms (12), and having a tempering device (51) for heating the preforms (12), and having a sterilising device (52), wherein the tempering device (51) comprises a microwave irradiation device (24) and the sterilising device (52) is at least partly integrated into the handling device (10), **characterised in that** the handling devices (10) each have holding awls (32) for holding the preforms (12) at their neck section (33), which holding awls (32) are provided with nozzles (36) for introducing a sterilising medium.

9. The blow moulding machine (54) as recited in claim 8, **characterised in that** the nozzles (36) of the holding awls (32) are each impingeable with sterilising medium via supply and/or discharge lines (38), and **in that** the sterilising medium can be extracted by suction.

10. The blow moulding machine (54) as recited in one of the claims 8 to 9, **characterised in that** the blow moulding machine (54) is constructed as a rotary machine and/or as a stretch blow moulding machine, in which the preforms (12) are continuously formable into sterilised containers.

## Revendications

1. Procédé de prétraitement de préformes (12) en matière plastique thermoplastique avant que celles-ci soient transformées en récipients au moyen d'un procédé de soufflage, dans lequel procédé les préformes (12) sont tempérées et stérilisées pour le procédé de soufflage subséquent, une thermorégulation des préformes (12) étant réalisée au moyen d'un traitement par micro-ondes, **caractérisé par** une stérilisation des préformes en introduisant un milieu de stérilisation liquide et/ou gazeux, la stérilisation des préformes (12) se faisant avant et/ou durant l'irradiation par micro-ondes et le milieu de stérilisation étant évaporé au moyen de l'irradiation par micro-ondes.

2. Procédé selon la revendication 1, **caractérisé par le fait que** le milieu de stérilisation comprend du peroxyde d'hydrogène (H₂O₂) liquide et/ou gazeux et/ou de l'acide paracétique.

3. Procédé selon l'une quelconque des revendications 1 ou 2, **caractérisé par le fait que** l'on utilise en tant que milieu de stérilisation un mélange d'air chaud et de peroxyde d'hydrogène et/ou de l'acide paracétique.

4. Procédé selon l'une quelconque des revendications 1 à 3, **caractérisé par le fait que** le milieu de stérilisation est introduit dans la préforme (12) au moyen d'au moins une buse (36) respectivement.

5. Procédé selon l'une quelconque des revendications 1 à 4, **caractérisé par le fait que** les préformes (12) sont rincées au moyen d'un milieu de rinçage approprié et sont ainsi débarrassées de traces du milieu de stérilisation.

6. Procédé selon l'une quelconque des revendications 1 à 5, **caractérisé par le fait que** le chauffage et la stérilisation des préformes (12) est réalisé(e) dans un processus continu, en particulier sur une machine rotative.

7. Procédé selon l'une ou plusieurs des revendications 1 à 6, dans lequel la préforme (12) est maintenue, durant son prétraitement, par le biais d'une unité de maintien de préforme (32) qui pénètre dans une bouche d'une zone de col (33) de la préforme (12).

8. Machine de soufflage (54) destiné au prétraitement et à la transformation par soufflage de préformes (12) en matière plastique thermoplastique en récipients suivant le procédé selon les revendications 1 à 7, comprenant des dispositifs de manipulation (10) pour manipuler et pour traiter et transporter de façon continue les préformes (12), un dispositif de thermorégulation (51) pour chauffer les préformes (12) ainsi qu'un dispositif de stérilisation (52), ledit dispositif de thermorégulation (51) comprenant une unité d'irradiation par micro-ondes (24) et ledit dispositif de stérilisation (52) étant intégré au moins en partie audit dispositif de manipulation (10), **caractérisée par le fait que** les dispositifs de manipulation (10) présentent chacun des mandrins de maintien (32) destinés à maintenir les préformes (12) sur leur zone de col (33) et pourvus de buses (36) d'introduction d'un milieu de stérilisation.

9. Machine de soufflage (54) selon la revendication 8, **caractérisée par le fait que** les buses (36) des mandrins de maintien (32) peuvent être alimentées chacune en milieu de stérilisation via des conduites d'alimentation et/ou d'évacuation (38), et que celui-ci peut être aspiré.

10. Machine de soufflage (54) selon l'une quelconque des revendications 8 à 9, **caractérisée par le fait que** la machine de soufflage (54) est réalisée en tant que machine rotative et/ou en tant que machine d'étirage-soufflage dans laquelle les préformes (12) peuvent être transformées de manière continue en récipients stérilisés.
